# FASCICULE DE BREVET EUROPEEN

(11) **EP 1 755 469 B1**
(45) Date de publication et mention de la délivrance du brevet: **07.10.2009**
(21) Numéro de dépôt: 05775312.1
(22) Date de dépôt: 07.06.2005
(51) Int. Cl.: A61B 17/32

(54) **DISPOSITIF POUR PRELEVEMENT DE GREFFONS CAPILLAIRES DE PETIT DIAMETRE**
VERFAHREN ZUR ENTNAHME VON HAARTRANSPLANTATEN MIT KLEINEM DURCHMESSER
DEVICE FOR COLLECTING SMALL DIAMETER HAIR GRAFTS

(30) Priorité: 14.06.2004 FR 0451169
(43) Date de publication de la demande: 28.02.2007
(73) Titulaire: Société de Promotion et Diffusion d'Equipment Médical Medicamat, 92240 Malakoff (FR)
(72) Inventeur: CORRE, Alain, F-75011 Paris (FR)
(74) Mandataire: Busnel, Jean-Benoît
(86) Numéro de dépôt international: PCT/FR2005/001390
(87) Numéro de publication internationale: WO 2006/003296

(56) Documents cités:
- FR-A1- 2 696 334
- US-A- 4 605 011
- US-A- 5 197 968
- US-A- 5 871 454
- US-A- 6 027 512
- US-A1- 2002 058 945

## Description

L'invention concerne un dispositif pour prélèvement de greffons capillaires de petit diamètre.

En particulier, cette invention est destinée à être utilisée lors d'un traitement chirurgical de la calvitie par transplantation capillaire qui consiste à transplanter, chez un même individu, des racines d'une partie de la chevelure située par exemple dans la couronne (toujours chevelue), vers la zone chauve.

Pour ce faire, on connaît la méthode dite des bandelettes, qui consiste à découper une ou plusieurs bandes de peau dans la zone par exemple de la couronne, puis à étirer ces bandes de peau de sorte à séparer distinctement les cheveux les uns des autres, à découper des fragments, appelés greffons, dans ces bandes de peau puis enfin à transplanter ces greffons sur la zone chauve préalablement préparée.

Cette méthode présente plusieurs inconvénients. Tout d'abord, avant la transplantation, un nombre important d'étapes doit être mis en oeuvre, qui impliquent des manipulations fastidieuses et nécessitent un temps opératoire important. En outre, cette méthode est douloureuse pour l'individu concerné, et la zone sur laquelle les bandes de peau ont été prélevées ne retrouve jamais son aspect normal puisque la découpe laisse une cicatrice de 15 à 20 cm de long.

On connaît également la technique dite des punchs qui consiste à découper et à prélever des fragments cutanés cylindriques de faible diamètre, appelés aussi greffons, typiquement de l'ordre de 0,7mm à 2mm, chaque fragment comprenant 1 ou 2 cheveux (micro-greffon) ou 3 à 5 cheveux (mini-greffon), puis à réimplanter ces greffons un par un dans les sites receveurs respectifs préalablement préparés. Cette technique présente l'avantage de ne pas être invalidante pour l'individu concerné, le faible diamètre des greffons prélevés permettant à la peau de se reconstituer très rapidement.

Pour mettre en oeuvre cette technique, on connaît du document FR-A-2 696 334 un dispositif pour transplantation comprenant un instrument portatif de coupe comportant un corps porte-outil, un outil cylindrique rotatif formé d'une aiguille creuse appelée punch et des moyens de prélèvement du greffon par aspiration dudit greffon au travers de l'aiguille.

Ce dispositif comporte par ailleurs, un appareil portatif d'implantation du greffon prélevé comprenant un corps, un réservoir de stockage intermédiaire du greffon, une aiguille creuse de réimplantation, et des moyens d'introduction du greffon dans le cuir chevelu au travers de l'aiguille creuse de réimplantation, à partir du réservoir intermédiaire.

Dans ce dispositif, le réservoir de stockage intermédiaire du greffon fait partie intégrante de l'appareil d'implantation, ce qui implique que chaque greffon, une fois découpé, passe non seulement à travers l'instrument de coupe, mais également à travers le tuyau reliant l'instrument de coupe et l'appareil d'implantation, ce qui présente plusieurs inconvénients.

Tout d'abord, le risque d'obturation dudit tuyau par les greffons est élevé, étant donné que la longueur de ce tuyau est importante. En outre, la circulation des greffons dans le tuyau est susceptible de provoquer leur altération, les greffons étant constitués de matière organique, et étant en conséquence particulièrement fragiles. Egalement, le fait que le réservoir de stockage soit situé à l'intérieur de l'appareil d'implantation empêche l'opérateur de visualiser correctement le greffon et par conséquent d'apprécier sa qualité avant son implantation sur la zone chauve de l'individu concerné. De plus, la simultanéité du prélèvement et de l'implantation demande une très bonne coordination des deux opérateurs.

Pour pallier ces inconvénients, l'invention propose un dispositif pour prélèvement de greffons capillaires de petit diamètre dans lequel un réservoir de stockage de greffons est solidaire du corps de l'instrument portatif de coupe, ce qui permet non seulement d'éviter tout risque d'obturation de tuyaux mais également d'éviter toute altération des greffons après leur découpe. En outre, l'efficacité de l'aspiration desdits greffons est augmentée, le réservoir étant disposé entre l'extrémité d'une aiguille interne et les moyens d'aspiration. Un tel agencement présente donc des avantages non seulement en termes de qualité des greffons implantés, mais également en termes de rapidité du prélèvement qu'il permet de mettre en oeuvre.

A cet effet, l'invention propose un dispositif pour prélèvement de greffons capillaires de petit diamètre comprenant un instrument portatif de coupe, ledit instrument comprenant :
- un corps porte-outil ;
- un outil cylindrique à axe creux permettant l'insertion d'une aiguille creuse de diamètre variant entre 0,7 mm et 2 mm ;
- un moteur apte à entraîner ladite aiguille en rotation par rapport audit corps, ledit dispositif comprenant en outre un réservoir de stockage de greffons solidaire dudit corps, ledit réservoir comprenant un orifice d'entrée des greffons connecté au voisinage de l'extrémité arrière de l'aiguille creuse, et un orifice d'aspiration connecté à des moyens d'aspiration du greffon, lesdits moyens d'aspiration étant agencés pour permettre au greffon de déboucher directement dans le réservoir par aspiration dudit greffon au travers de l'aiguille.

Selon une réalisation, le corps porte-outil est une pièce à main de forme sensiblement cylindrique.

Selon une autre réalisation, le corps porte-outil est une pièce à main de type pièce à main à contre-angle. Cette réalisation présente notamment un avantage en terme de coût, une telle pièce à main pouvant être élaborée à partir d'appareils utilisés couramment, notamment dans le domaine de la chirurgie dentaire, et par conséquent disponibles commercialement. En outre, l'axe creux prévu ici pour l'insertion de l'aiguille et le passage des greffons par aspiration pourrait également être réalisé indépendamment du réservoir de stockage précité.

D'autres objets et avantages de l'invention apparaîtront au cours de la description qui suit, faite en référence aux dessins annexés dans lesquels :
- la figure 1 est une vue schématique en coupe longitudinale d'un dispositif selon l'invention, selon un premier mode de réalisation ;
- la figure 2 est une vue schématique en coupe longitudinale d'un dispositif selon l'invention, selon un deuxième mode de réalisation ;
- la figure 3 est une vue schématique en coupe longitudinale d'un dispositif selon l'invention, selon un troisième mode de réalisation.

Le dispositif selon l'invention est destiné au prélèvement de greffons capillaires de petit diamètre. Par petit diamètre, on entend un diamètre compris entre environ 0,7mm et 2mm.

Dans la description qui suit, on définit par « avant » le côté du dispositif destiné à être en contact avec la peau de l'individu sur lequel sont prélevés les greffons, et par arrière le côté opposé. Et on définit par « longitudinale » la direction avant - arrière du dispositif, et par « transversale » la direction perpendiculaire.

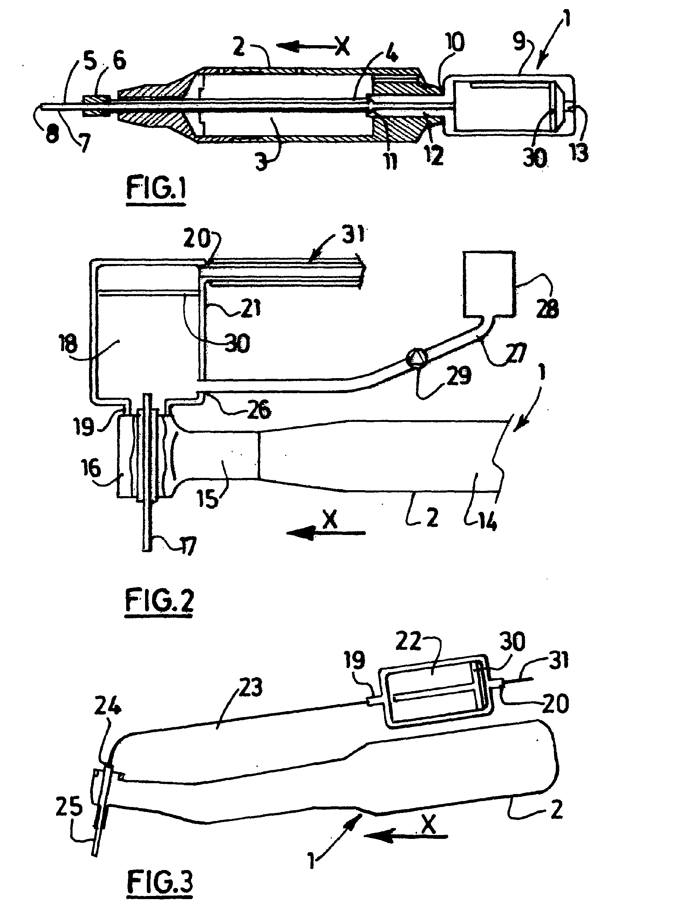

En référence à la figure 1, on décrit un premier mode de réalisation de ce dispositif.

Selon cette réalisation, l'instrument portatif de coupe 1 comprend un corps porte-outil 2 formé d'une pièce à main de forme sensiblement cylindrique dans lequel est disposé, selon un axe central longitudinal X, un outil cylindrique comprenant un moteur 3 à axe creux 4. Cet outil est agencé pour permettre, dans son axe creux 4, l'insertion d'une aiguille métallique 5 creuse, cylindrique, droite, de diamètre variant entre 0,7 mm et 2 mm, de sorte à permettre la circulation des greffons à l'intérieur de ladite aiguille. Une telle aiguille 5 est communément appelée « punch » dans le domaine de la transplantation capillaire.

L'aiguille 5 est agencée pour pouvoir être entraînée par le moteur 4 en rotation axiale par rapport audit corps 2.

L'aiguille 5 est maintenue dans l'axe creux 4 au moyen d'une liaison 6 disposée à l'avant et à l'extérieur du corps 2.

L'extrémité avant 7 de l'aiguille 5 dépasse de quelques centimètres à l'avant du corps 2. Cette extrémité 7 présente un bord circulaire 8 biseauté et tranchant, de sorte à permettre la découpe du greffon.

Le dispositif comprend en outre un réservoir de stockage 9 de greffons solidaire du corps 2, formé d'un flacon de forme sensiblement cylindrique. Le réservoir 9 comprend un orifice d'entrée 10 des greffons connecté au voisinage de l'extrémité arrière 11 de l'aiguille 5 via un connecteur 12. Dans le mode de réalisation représenté, ce connecteur 12 fait partie intégrante du réservoir 9. Toutefois, on peut également prévoir que le connecteur soit une pièce séparée.

Le réservoir 9 comprend un orifice d'aspiration 13 connecté à des moyens d'aspiration, ces moyens d'aspiration étant agencés pour permettre au greffon de déboucher directement dans le réservoir 9, chaque greffon, une fois découpé, étant aspiré au travers de l'aiguille 5 puis de l'axe central du connecteur 12.

Selon ce mode de réalisation, l'orifice d'entrée 10 des greffons et l'orifice d'aspiration 13 sont disposés en regard l'un de l'autre, ce qui favorise l'efficacité de l'aspiration desdits greffons.

Selon le deuxième mode de réalisation, représenté sur la figure 2, le corps porte-outil 2 est une pièce à main de type pièce à main à contre-angle. Une telle pièce à main comprend un manche 14, un col 15, et une tête 16 dans laquelle est insérée une aiguille creuse 17.

Le fait d'utiliser une telle pièce à main à contre-angle, dont la tête 16 comprend un axe central creux, présente l'avantage de conférer à l'opérateur un meilleur champ de vision de la zone de prélèvement de chaque greffon. En effet, la visibilité n'est pas gênée par le col 15 et le manche 14 de la pièce à main, puisque ceux-ci forment un angle avec la tête 16 et, en outre, la tête est de taille relativement faible, du fait que le moteur d'entraînement de l'aiguille 17 soit situé dans le manche 14 de la pièce.

Le réservoir de stockage correspondant 18 est connecté au voisinage de l'extrémité arrière de l'aiguille 17, ladite extrémité arrière débouchant sensiblement au centre de l'orifice d'entrée des greffons 19. Ainsi, chaque greffon, une fois découpé, entre directement dans le réservoir 18 après avoir circulé par aspiration à travers l'aiguille 17. L'orifice d'aspiration 20 débouche ici sur une paroi latérale 21 du réservoir 18.

Selon le troisième mode de réalisation, représenté sur la figure 3, un réservoir 22 est solidarisé au corps 2 par l'intermédiaire d'un conduit flexible 23 de longueur sensiblement inférieure ou égale à la longueur du corps 2, ledit conduit 23 étant connecté à l'extrémité arrière 24 d'une aiguille creuse 25. Cet agencement est représenté avec une pièce à main de type pièce à main à contre-angle, similaire à celle décrite en relation avec le deuxième mode de réalisation.

Cet agencement présente l'avantage d'améliorer encore, pour l'opérateur, la visibilité de la zone de prélèvement de chaque greffon, puisque le réservoir 22 est situé à distance de la tête de la pièce à main, et sans en être pour autant trop éloigné, afin de préserver la bonne maniabilité du dispositif. En outre, il n'est pas nécessaire, selon cette réalisation, de prévoir un réservoir dont la taille est ajustée à celle de la tête, tel que représenté dans la figure 2. On peut ainsi envisager par exemple un réservoir 22 de taille supérieure à celui représenté sur la figure 2, susceptible de recevoir ainsi un plus grand nombre de greffons.

En outre, les aiguilles 17, 25 représentées sur les figures 2 et 3 sont courtes, ce qui présente l'avantage de permettre une meilleure aspiration, en particulier pour les greffons de faible diamètre, ainsi qu'une meilleure concentricité d'où une meilleure coupe.

Selon une réalisation particulière, la solidarisation du réservoir 9, 18, 22 sur le corps 2 est réalisée par des moyens de solidarisation étanches, de type connu, pour éviter notamment toute pénétration d'air susceptible de nuire au processus d'aspiration du greffon.

Par ailleurs, on prévoit que l'aiguille 5, 17, 25 soit montée amovible par rapport au corps 2, ce qui facilite son entretien ou peut permettre son changement à intervalles réguliers.

Egalement, et tel que représenté sur la figure 2, le réservoir peut comprendre un orifice d'entrée 26 d'une solution de conservation de greffons, ledit orifice 26 étant connecté à un conduit d'alimentation 27 en solution de conservation, l'alimentation étant réalisée depuis un flacon 28 contenant ladite solution par exemple par gravité ou vacuum ou au moyen d'une pompe péristaltique 29.

En particulier, cette solution de conservation comprend du sérum physiologique, qui permet de conserver et d'humidifier les greffons contenus dans le réservoir. On peut également envisager d'utiliser, dans le sérum physiologique, des additifs permettant par exemple d'améliorer cette conservation.

Par ailleurs, selon les modes de réalisation représentés, le réservoir 9, 18, 22 comprend en outre un filtre 30 qui s'étend sur toute la section transversale dudit réservoir, au voisinage de l'orifice d'aspiration. La présence d'un tel filtre permet de retenir les greffons dans le réservoir.

Les moyens d'aspiration auxquels est connecté l'orifice d'aspiration du réservoir 9, 18, 22 peuvent comprendre une pompe à vide et des moyens de contrôle et de mesure du vide, la connexion étant réalisée au moyen d'un conduit 31.

En outre, on peut prévoir que les parois du réservoir de stockage 9, 18, 22 soient transparentes, ce qui permet à l'opérateur de vérifier la qualité des greffons au fur et à mesure de leur arrivée dans le réservoir, et de vérifier que leur angle d'obtention lors du prélèvement est satisfaisant.

## Revendications

1. Dispositif pour prélèvement de greffons capillaires de petit diamètre comprenant un instrument portatif de coupe (1), ledit instrument comprenant :
- un corps porte-outil (2) ;
- un outil cylindrique à axe creux (4) ;
- une aiguille creuse (5, 17, 25) de diamètre variant entre 0,7 mm et 2 mm insérée dans l'axe creux (4) de l'outil cylindrique ;
- un moteur (3) apte à entraîner ladite aiguille (5, 17, 25) en rotation par rapport audit corps (2) ;
- des moyens d'aspiration de greffons agencés pour permettre au greffon de déboucher directement dans le réservoir (9, 18, 22) par aspiration dudit greffon au travers de l'aiguille (5, 17, 25). ;
- un réservoir de stockage (9, 18, 22) de greffons comprenant un orifice d'aspiration (13, 20) connecté à des moyens d'aspiration du greffon ; et
ledit dispositif étant **caractérisé en ce que** le réservoir de stockage (9, 18, 22) de greffons est solidaire dudit corps (2) et comprend un orifice d'entrée (10, 19) des greffons connecté au voisinage de l'extrémité arrière de l'aiguille creuse (5, 17, 25), l'extrémité arrière de l'aiguille creuse (5, 17, 25) débouchant au centre de l'orifice d'entrée (10, 19) des greffons

2. Dispositif selon la revendication 1, **caractérisé en ce que** le corps porte-outil (2) est une pièce à main de forme sensiblement cylindrique.

3. Dispositif selon la revendication 1, **caractérisé en ce que** le corps porte-outil (2) est une pièce à main de type pièce à main à contre-angle.

4. Dispositif selon l'une quelconque des revendications 1 à 3, **caractérisé en ce qu'**il comprend des moyens de solidarisation étanches dudit réservoir (9, 18, 22) sur ledit corps (2).

5. Dispositif selon l'une quelconque des revendications 1 à 4, **caractérisé en ce que** l'aiguille creuse (5, 17, 25) est montée amovible par rapport au corps (2).

6. Dispositif selon l'une quelconque des revendications 1 à 5, **caractérisé en ce que** le réservoir de stockage (9, 18, 22) comprend un filtre (30), ledit filtre s'étendant sur toute la section transversale dudit réservoir, au voisinage de l'orifice d'aspiration (13, 20).

7. Dispositif selon l'une quelconque des revendications 1 à 6, **caractérisé en ce que** le réservoir (9, 18, 22) comprend en outre un orifice d'entrée (26) d'une solution de conservation de greffons, ledit orifice étant connecté à un conduit d'alimentation (27) en solution de conservation.

8. Dispositif selon la revendication 7, **caractérisé en ce que** la solution de conservation comprend du sérum physiologique.

9. Dispositif selon l'une quelconque des revendications 1 à 8, **caractérisé en ce que** les parois du réservoir de stockage (9, 18, 22) sont transparentes.

## Claims

1. A device for sampling small diameter capillary grafts including a portable cutting instrument (1), said instrument including:
- a tool-holder body (2);
- a hollow axis cylindrical tool (4);
- a hollow needle (5, 17, 25) having a diameter between 0.7mm and 2mm inserted into the hollow axis (4) of the cylindrical tool;
- a motor (3) capable of driving said needle (5, 17, 25) into rotation with respect to said body (2);
- graft suction devices so arranged as to enable the graft to be delivered directly into the tank (9, 18, 22), through the suction of said graft through the needle (5, 17, 25);
- a graft storage tank (9, 18, 22) including a suction hole (13, 20) connected to graft suction means; and
said device being **characterised in that** the graft storage tank (9, 18, 22) is integral with said body (2) and includes a graft inlet (10, 19) connected in the vicinity of the rear end of the punch needle (5, 17, 25), with the rear end of the punch needle (5, 17, 25) being connected at the centre of the graft inlet (10, 19).

2. A device according to claim 1, **characterised in that** the tool-holder body (2) is a hand-piece having a substantially cylindrical shape.

3. A device according to claim 1, **characterised in that** the tool-holder body (2) is a hand-piece of the counter-angle hand-piece type.

4. A device according to any one of claims 1 to 3, **characterised in that** it includes tight means for making said tank (9, 18, 22) integral with said body (2).

5. A device according to any one of claims 1 to 4, **characterised in that** the hollow needle (5, 17, 25) is mounted to be removable from the body (2).

6. A device according to any one of claims 1 to 5, **characterised in that** the storage tank (9, 18, 22) includes a filter (30), said filter extending on the whole cross-section of said tank, in the vicinity of the suction hole (13, 20).

7. A device according to any one of claims 1 to 6, **characterised in that** the tank (9, 18, 22) further includes an inlet (26) for a graft preservation solution, said hole being connected to a preservation solution supply pipe (27).

8. A device according to claim 7, **characterised in that** the preservation solution includes physiological serum.

9. A device according to any one of claims 1 to 8, **characterised in that** the walls of the storage tank (9, 18, 22) are transparent.

## Patentansprüche

1. Vorrichtung zur Entnahme von Haarimplantaten mit kleinem Durchmesser, die ein tragbares Schneidinstrument (1) umfasst, wobei das genannte Instrument Folgendes umfasst:
- einen Werkzeugträgerkörper (2);
- ein zylindrisches Werkzeug mit hohler Achse (4);
- eine hohle Nadel (5, 17, 25) mit einem zwischen 0,7 mm und 2 mm variierenden Durchmesser, die in der hohlen Achse (4) des zylindrischen Werkzeugs eingefügt ist;
- einen Motor (3), der geeignet ist, die genannte Nadel (5, 17, 25) im Verhältnis zu dem genannten Körper (2) in Rotation anzutreiben;
- Implantatansaugmittel, die angeordnet sind, um es dem Implantat zu ermöglichen, durch Ansaugen des genannten Implantats durch die Nadel (5, 17, 25) direkt in das Reservoir (9, 18, 22) zu münden;
- ein Lagerungsreservoir (9, 18, 22) für Implantate, das eine Ansaugöffnung (13, 20) umfasst, die an Ansaugmittel des Implantats angeschlossen ist; und wobei die genannte Vorrichtung **dadurch gekennzeichnet ist, dass** das Lagerungsreservoir (9, 18, 22) für Implantate fest mit dem genannten Körper (2) befestigt ist und eine Eingangsöffnung (10, 19) der Implantate umfasst, die in der Nachbarschaft des hinteren Endes der hohlen Nadel (5, 17, 25) angeschlossen ist, wobei das hintere Ende der hohlen Nadel (5, 17, 25) im Zentrum der Eingangsöffnung (10, 19) der Implantate mündet.

2. Vorrichtung gemäß Anspruch 1, **dadurch gekennzeichnet, dass** der Werkzeugträgerkörper (2) ein Handteil in deutlich zylindrischer Form ist.

3. Vorrichtung gemäß Anspruch 1, **dadurch gekennzeichnet, dass** der Werkzeugträgerkörper (2) ein Handstück vom Typ Handstück mit Gegenwinkel ist.

4. Vorrichtung gemäß Anspruch 1 bis 3, **dadurch gekennzeichnet, dass** sie Mittel zur festen, abgedichteten Befestigung des genannten Reservoirs (9, 18, 22) auf dem genannten Körper (2) umfasst.

5. Vorrichtung gemäß Anspruch 1 bis 4, **dadurch gekennzeichnet, dass** die hohle Nadel (5, 17, 25) im Verhältnis zum Körper (2) abnehmbar angebracht ist.

6. Vorrichtung gemäß Anspruch 1 bis 5, **dadurch gekennzeichnet, dass** das Lagerungsreservoir (9, 18, 22) einen Filter (30) umfasst, wobei sich der genannte Filter auf den gesamten transversalen Querschnitt des genannten Reservoir in der Nachbarschaft der Ansaugöffnung (13, 20) erstreckt.

7. Vorrichtung gemäß Anspruch 1 bis 6, **dadurch gekennzeichnet, dass** das Reservoir (9, 18, 22) darüber hinaus eine Eintrittsöffnung (26) einer Konservierungslösung für Implantaten umfasst, wobei die genannte Öffnung an eine Versorgungsleitung (27) für die Konservierungslösung angeschlossen ist.

8. Vorrichtung gemäß Anspruch 7, **dadurch gekennzeichnet, dass** die Konservierungslösung physiologisches Serum umfasst.

9. Vorrichtung gemäß Anspruch 1 bis 8, **dadurch gekennzeichnet, dass** die Wände des Lagerungsreservoirs (9, 18, 22) transparent sind.
